# EUROPEAN PATENT SPECIFICATION

(11) **EP 2 425 780 B1**
(45) Date of publication and mention of the grant of the patent: **08.01.2014**
(21) Application number: 10769364.0
(22) Date of filing: 23.04.2010
(51) Int. Cl.: A61B 6/04, A61G 13/12, A61N 5/10

(54) **DIAPHRAGM AND ABDOMINAL COMPRESSION SYSTEM**
DIAPHRAGMA UND ABDOMINALES KOMPRESSIONSSYSTEM
SYSTEME DE COMPRESSION DIAPHRAGMATIQUE ET ABDOMINALE

(30) Priority: 29.04.2009 ES 200930112
(43) Date of publication of application: 07.03.2012
(73) Proprietor: Servicio Andaluz de Salud, 41071 Sevilla (ES)
(72) Inventor: VELÁZQUEZ, Santiago, E-21005 Huelva (ES)
(74) Representative: Pons Ariño, Angel
(86) International application number: PCT/ES2010/070256
(87) International publication number: WO 2010/125218

(56) References cited:
- WO-A1-2005/034827
- WO-A2-2006/081412
- ES-T3- 2 181 719
- GB-A- 2 038 150
- US-A- 5 575 027
- US-A1- 2003 167 569
- US-A1- 2004 123 388
- US-A1- 2006 288 483
- US-A1- 2009 308 400
- US-B1- 6 826 423

## Description

### OBJECT OF THE INVENTION

The present invention pertains to the field of medicine, particularly describing a system to compress a patient's diaphragmatic and abdominal areas in order to prevent movement that may cause inaccuracies in the area being treated during procedures such as radiotherapy or others requiring the application of radiation to a specific tissue area.

### BACKGROUND OF THE INVENTION

The objective of external radiotherapy is the application of a tumoricidal dose to a pre-defined volume of tissue to destroy a tumour, usually being carried out through different fields of treatment with different incidences to achieve minimal irradiation of the surrounding healthy tissue and organs.

The arrival of conformal radiotherapy has led to an interesting qualitative change, achieving individualized irradiation of the volume to be treated, preserving a maximum of adjacent healthy structures. Nomenclature recommended by the ICRU 50 will be used in this document, which defines the gross target volume (GTV) as a region where a visible or tangible tumour exists, the clinical target volume (CTV) as a region potentially affected by a microscopic disease and the planning target volume (PTV) as the margin around the CTV which makes sure the CTV receives the prescribed radiation dose when considering errors in positioning and patient immobilization as well as internal organ movement.

Conformal radiotherapy has caused the PTV margin to acquire great importance, demonstrating that the selection of an insufficient or excessive margin can cause a decrease in tumour control and an increase in the toxicity due to damage to surrounding structures, respectively.

Thus, it becomes necessary to immobilize the patient with the object of achieving the maximum possible precision regarding the tissue being treated. However, even through a correct immobilization and the meticulous daily positioning of the patient receiving treatment, factors that may limit the treatment's success still exist, with the geometrical uncertainties surrounding the patient and his internal organs being a current theme of investigation.

These uncertainties may be divided in daily positioning errors, transfer errors and internal organ movements.
- Daily positioning errors are the variations of a patient's placement from one day to the other (lack of cooperation, muscle contractions, discomfort, etc.) with the corresponding lack of treatment precision.
- Transfer errors are those produced when going from a simulation TAC to the treatment unit.
- Organ movement includes physiologically produced motions and oscillations (breathing, peristaltic, bladder or rectum filling, etc.) causing vitally important positional variations when trying to receive a lower or higher amount of radiation.

Furthermore, treatment of areas near the diaphragm and abdominal area involve an added difficulty because respiratory movement makes the margin of error considerably large. Up to now, systems applying a single point of pressure for the reduction of lung movement have been employed. However, the drastic reduction in thoracic movement of these systems results in only a small percentage of patients being capable of tolerating its application.

For example, WO 2006081412 discloses a diaphragmatic and abdominal compression system according to the preamble of claim 1.

### DESCRIPTION OF THE INVENTION

The present invention describes a diaphragmatic and abdominal compression system that diminishes lower thoracic and abdominal organ movement, thus reducing radiation's margin of error due to patient movement, and, as a result, the volume of healthy tissue being irradiated. This way, the fundamental objective of the radiotherapy treatment may be achieved, which is the CTV's correct irradiation, respecting, at the same time, the tolerance dose to radiation of surrounding tissues and organs, which are frequently located in areas adjacent to the CTV.

The diaphragmatic compression system of the invention increases the number of pressure points applied to the patient, some of which may also have a variable aim angle. This configuration causes a qualitative change in immobilization performance, and it also allows compression of a single area of interest, such as the lateral abdominal area and the retro abdominal area, so that patients who do not tolerate the complete thoracic immobilization from the previous technique may benefit from this new system.

In this document, the term "protuberance" will be used to make reference to the pressure points applied to the patient. It is understood that each protuberance comprises means allowing its axial movement to accommodate them to each particular patient's volume. In addition, the shape of the protuberances' end making contact with the patient must be adequate for this purpose, i.e. must prevent damage to the patient due to sharp edges, aggressive materials, etc.

Thus, one aspect of the invention describes a diaphragmatic and abdominal compression system to immobilize a patient's diaphragmatic and abdominal area which comprises a support structure from which two rear protuberances project, designed to compress a patient's renal areas, and two side protuberances designed to compress over or below the patient's lowermost floating ribs and further comprising an upper arch attached to the support structure which supports two front protuberances designed to be supported on the front part of the patient's thoracic cage. The rear protuberances compress the patient's renal area in order to achieve a reduction in kidney movement inside the perirenal fat capsule, while the side protuberances diminish diaphragmatic movement due to breathing.

The support structure has the appropriate shape and dimensions to accommodate a patient lying on his back. In addition, it must be as rigid as possible to contain the stress to which it will be put through by the patient's diaphragmatic muscles. In preferred embodiments of the invention, a flexible mold, made of, for example polystyrene, is placed over the area where the rear and side protuberances are found so that the patient is as comfortable as possible. In another preferred embodiment of the invention, the support structure comprises a horizontal lower plate and a couple of vertical side plates as rails.

The upper arch may preferably support a central protuberance designed to be supported under the patient's sternum. This upper arch may have any shape as long as it supplies a rigid support to said front and central protuberances.

It is also possible to design the upper arch in a way that it may pivot in relation to an axis transversal to the patient, contained in the coronal plane so that the aim angle of the frontal protuberances and, in its case, of the central protuberance regarding the patient's thoracic cage, may be variable. This way, it is possible to precisely regulate the point and angle of application of the central protuberance, which diminishes the longitudinal movement within a coronal plane of the thoracic cage during breathing. In addition, the support structure may comprise attachment means to couple the rear and side protuberances and the upper arch in different positions.

Another preferred embodiment of the invention comprises a handling structure attachable to the support structure in different positions next to the end corresponding to the patient's head. The handling structure serves to provide the patient a handle allowing him to easily maintain the position, as well as keeping the patient's arms far from the treatment area.

Finally, preferred embodiments of the diaphragmatic compression system's support structure of the invention comprise, respectively, of a radiopaque ruler, of the kind, for example, used in radiology, and gaps designed for the installation of fiducial markers in PET, SPECT or MRI treatments.

### BRIEF DESCRIPTION OF THE DRAWINGS

Fig. 1 shows an upper-front view in perspective of a diaphragmatic compression system comprising an upper arch.
Fig. 2 shows a front view of the system in Fig. 1 with the flexible mold positioned.
Figs. 3a and 3b show a protuberance sample according to the invention.
Fig. 4 shows a perspective view of an immobilized patient using the system in figures 1 and 2.
Fig. 5 shows a second perspective view of an immobilized patient with the system of the invention.

### PREFERRED EMBODIMENT OF THE INVENTION

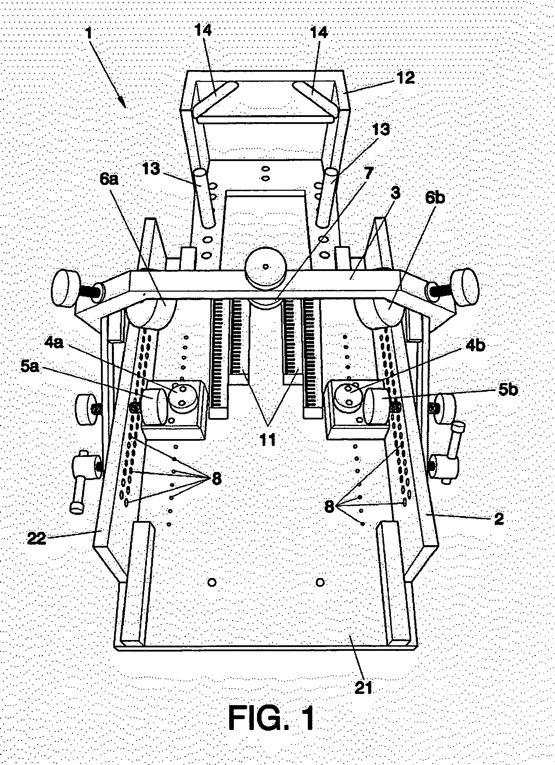

Described below is an embodiment of the present invention making reference to the attached figures. Fig. 1 shows an embodiment of the system (1) according to the present invention wherein the support structure (2) can be seen, which, in this embodiment, comprises a horizontal plate (21) and a couple of vertical side plates (22) to which an upper arch (3) is attached. The attachment points of the upper arch (3) to the support structure (2) allow detaching said upper arch (3), as well as its rotation around an axis transversal to the patient and contained in a coronal plane. This allows selecting the aim angle of the front protuberances (6a, 6b), and the central protuberance (7) for the best adaptation possible to the patient. The connection between the upper arch (3) and the support structure (2) and, in general, all connections between the different parts of the invention's system must be sufficiently rigid so as to sustain the stress to which they will be subjected during use.

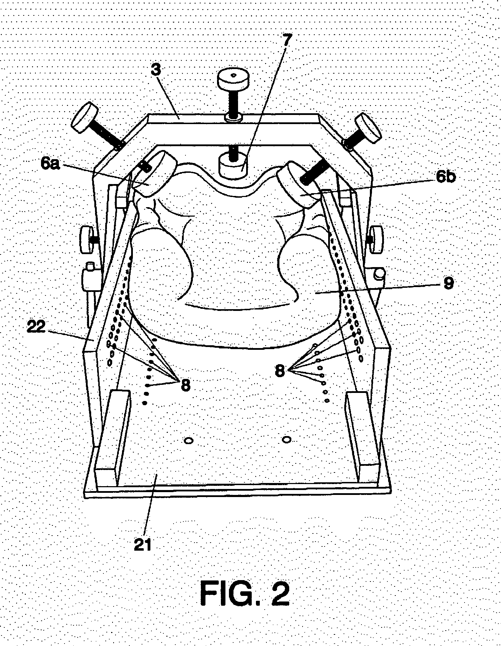

Also, the rear protuberances (4a, 4b), side protuberances (5a, 5b) front protuberances (6a, 6b) and the central protuberance (7) can be seen. In this embodiment, the rear protuberances (4a, 4b) are fixed, with a total height of 70 mm, which normally is the maximum compression tolerated by a patient. Fig. 2 also shows a flexible mold (9) made of polystyrene spheres and hydraulic resin in this embodiment, which improves patient immobilization while at the same time improving comfort.

The handling structure (12) comprises some tholes (13) that serve to give shape to the flexible mold and provide maximum comfort to the patient, in addition to handles (14). The tholes (13) may be arranged in different positions depending on the patient's height and volume.

In addition, a radiopaque ruler (11) fixed over the horizontal plate (21) can be seen in the figures, as well as a plurality of attachment means (8), in this example threaded holes allowing the upper arch (3) and the back protuberances (4a, 4b) and the side protuberances (5a, 5b) to be attached in different positions along the support structure (2) and also at different heights. Furthermore, the detachable upper arch (3) allows freeing the space above the support structure (2) to allow the patient to be placed more comfortably in the diaphragmatic compression system (1) of the invention.

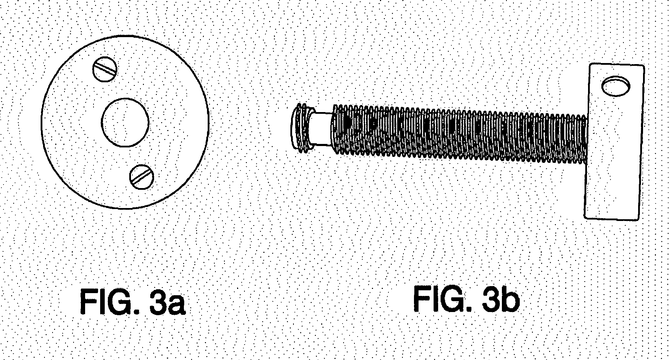

In this embodiment, the protuberances (4a, 4b, 5a, 5b, 6a, 6b, 7) are formed by a screw or rod, represented in Fig. 3b, one of whose ends comprise an operation head, while the free end gets coupled to a cap, such as the one represented on Fig. 3a, which will be the part making contact with the patient's skin. The cap is adapted in this embodiment in a way that it is not drawn by the rod's rotation during its axial movement produced by the rotation of the operation head. This allows adjusting the height of each protuberance (4a, 4b, 5a, 5b, 6a, 6b, 7) without producing uncomfortable friction on the patient's skin. Both the rod and the cap of this example are made of nylon.

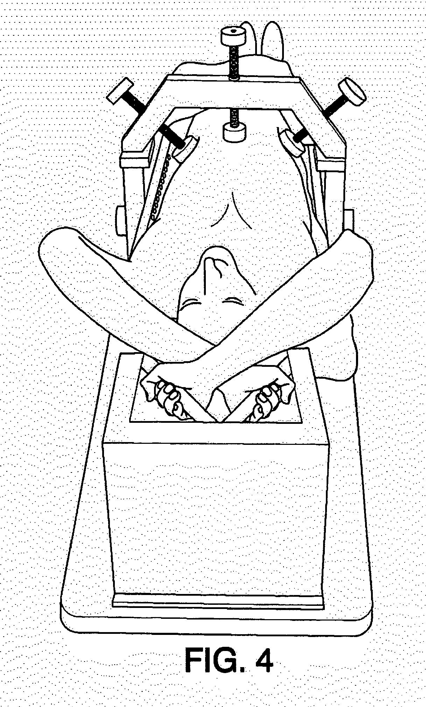

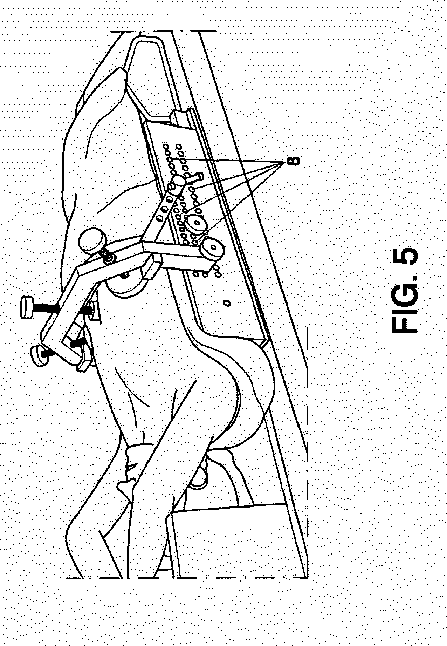

Figs. 4 and 5 show a patient immobilized by the diaphragmatic compression system (1) of the invention. First, the patient lies on the support structure (2) to which the rear protuberances (4a, 4b) and side protuberances (5a, 5b) have been previously attached in a way that they respectively coincide with the patient's lumbar area and lowermost floating rib, and where a flexible mold (9) has also been placed. Next, the upper arch (3) is attached in the appropriate position and height and the front protuberances (6a, 6b) and the central protuberances (7) are axially displaced until they respectively, firmly press the thoracic area and the area located under the patient's sternum. The patient remains, this way, immobilized, enormously improving the performance of devices based on a single-point pressure application.

It can also be seen that the figure's system (1) in addition to the parts described in this document, comprises an additional structure (10) where the patient rests his head and arms during treatment. This additional structure (10) may also comprise supports to give the flexible mold (9) the most adequate shape for each case.

## Claims

1. Diaphragmatic and abdominal compression system (1) for immobilizing the thoracic and abdominal areas of a patient, **characterized in that** it comprises a support structure (2) from which two rear protuberances (4a, 4b) designed to compress the patient's renal areas, and two side protuberances (5a, 5b) designed to compress over or below the patient's lowermost floating ribs, project, and further comprising an upper arch (3) attached to the support structure (2) which supports two front protuberances (6a, 6b) designed to be supported on the front part of the patient's thoracic cage.

2. Diaphragmatic and abdominal compression system (1) according to claim 1, wherein the upper arch (3) also supports a central protuberance (7) designed to be supported under the patient's sternum.

3. Diaphragmatic and abdominal compression system (1) according claim 2, wherein the aim angle of the front protuberances (6a, 6b) and central protuberance (7) is variable.

4. Diaphragmatic and abdominal compression system (1) according to any of the previous claims wherein the support structure (2) comprises attachment means (8) for attaching the back protuberances (4a, 4b), the side protuberances (5a, 5b) and the upper arch (3) in different positions.

5. Diaphragmatic and abdominal compression system (1), according to any of the previous claims, wherein the support structure (2) further comprises a radiopaque ruler (11).

6. Diaphragmatic and abdominal compression system (1) according to any of the previous claims, wherein the support structure (2) further comprises gaps for the installation of fiducial markers.

7. Diaphragmatic and abdominal compression system (1) according to any of the previous claims, further comprising a flexible mold (9) placed over the support structure (2).

8. Diaphragmatic and abdominal compression system (1) according to claim 2, wherein the protuberances (4a, 4b, 5a, 5b, 5a, 5b, 7) are formed by a screw having an end coupled to a cap for making contact with the patient's skin, said cap being adapted not to be drawn by the rotation of the screw.

9. Diaphragmatic and abdominal compression system (1) according to any of the previous claims, further comprising a handling structure (12) attachable to the support structure (2) for providing the patient with a handle.

## Patentansprüche

1. System zur Kompression des Zwerchfells und Bauchraums (1) zwecks Immobilisierung des Brustkorb- und Bauchraumbereichs eines Patienten, **dadurch gekennzeichnet, dass** dieses eine Stützstruktur (2) umfasst, aus der zwei hintere Vorsprünge (4a, 4b) herausragen, die dazu ausgelegt sind, den Nierenbereich eines Patienten zu komprimieren, sowie zwei seitliche Vorsprünge (5a, 5b), die dazu ausgelegt sind, den Bereich über oder unter den untersten freien Rippen des Patienten zu komprimieren, wobei dieses weiterhin einen oberen Bogen (3) umfasst, der an der Stützstruktur (2) befestigt ist, welche die beiden vorderen Vorsprünge (6a, 6b) stützt, die dazu ausgelegt sind, auf dem vorderen Teil des Brustkorbs des Patienten aufgestützt zu werden.

2. System zur Kompression des Zwerchfells und Bauchraums (1) nach Anspruch 1, bei dem der obere Bogen (3) ebenfalls einen mittigen Vorsprung (7) stützt, der dazu ausgelegt ist, unter dem Brustbein des Patienten aufgestützt zu werden.

3. System zur Kompression des Zwerchfells und Bauchraums (1) nach Anspruch 2, bei dem der Zielwinkel der vorderen Vorsprünge (6a, 6b) und des mittigen Vorsprungs (7) variabel ist.

4. System zur Kompression des Zwerchfells und Bauchraums (1) nach einem der vorherigen Ansprüche, bei dem die Stützstruktur (2) Befestigungsmittel (8) zur Befestigung der hinteren Vorsprünge (4a, 4b), der seitlichen Vorsprünge (5a, 5b) und des oberen Bogens (3) in unterschiedlichen Positionen umfasst.

5. System zur Kompression des Zwerchfells und Bauchraums (1) nach einem der vorherigen Ansprüche, bei dem die Stützstruktur (2) weiterhin ein röntgendichtes Lineal (11) umfasst.

6. System zur Kompression des Zwerchfells und Bauchraums (1) nach einem der vorherigen Ansprüche, bei dem die Stützstruktur (2) weiterhin Lücken für die Anbringung von Referenzmarkierern umfasst.

7. System zur Kompression des Zwerchfells und Bauchraums (1) nach einem der vorherigen Ansprüche, das weiterhin eine flexible Form (9) umfasst, die über der Stützstruktur (2) platziert ist.

8. System zur Kompression des Zwerchfells und Bauchraums (1) nach Anspruch 2, bei dem die Vorsprünge (4a, 4b, 5a, 5b, 5a, 5b, 7) von einer Schraube gebildet werden, bei der ein Ende mit einer Kappe verbunden ist, um die Haut des Patienten zu berühren, wobei die Kappe dazu ausgelegt ist, nicht durch die Drehung der Schraube mitgezogen zu werden.

9. System zur Kompression des Zwerchfells und Bauchraums (1) nach einem der vorherigen Ansprüche, das weiterhin eine Handhabungsstruktur (12) umfasst, die an der Stützstruktur (2) befestigt werden kann, um dem Patienten einen Handgriff bereitzustellen.

## Revendications

1. Un système de compression diaphragmatique et abdominale (1) permettant d'immobiliser les zones thoracique et abdominale du patient, **caractérisé en ce qu'**il dispose d'une structure portante (2) de laquelle sortent deux protubérances arrières (4a, 4b) destinées à comprimer les zones rénales du patient, de deux protubérances latérales (5a, 5b) destinées à comprimer par-dessus et par-dessous les côtes flottantes inférieures du patient et dispose également d'un arc supérieur (3) rattaché à la structure portante (2) munie de deux protubérances avant (6a, 6b) destinées à être soutenues à l'avant de la cage thoracique du patient.

2. Un système de compression diaphragmatique et abdominale (1) selon la revendication 1, dont l'arc supérieur (3) est également muni d'une protubérance centrale (7) destinées à être soutenue sous le sternum du patient.

3. Un système de compression diaphragmatique et abdominale (1) selon la revendication 2, dont l'angle de visée des protubérances avant (6a, 6b) et de la protubérance centrale (7) est variable.

4. Un système de compression diaphragmatique et abdominale (1) selon une des revendications précédentes, dont la structure portante (2) dispose de moyens de fixation permettant de fixer les protubérances arrières (4a, 4b), les protubérances latérales (5a, 5b) ainsi que l'arc supérieur (3) dans diverses positions.

5. Un système de compression diaphragmatique et abdominale (1) selon une des revendications précédentes, dont la structure portante (2) dispose également d'une règle radio-opaque (11).

6. Un système de compression diaphragmatique et abdominale (1) selon une des revendications précédentes, dont la structure portante (2) dispose également d'ouvertures permettant d'installer des repères de centrage.

7. Un système de compression diaphragmatique et abdominale (1) selon une des revendications précédentes, disposant également d'un moule flexible (9) placé sur la structure portante (2).

8. Un système de compression diaphragmatique et abdominale (1) selon la revendication 2, dont les protubérances (4a, 4b, 5a, 5b, 5a, 5b, 7) sont constituées d'une vis dont l'extrémité est couplée à un capuchon destiné à entrer en contact avec la peau du patient, ledit capuchon étant conçu de sorte à ne pas tourner avec la rotation de la vis.

9. Un système de compression diaphragmatique et abdominale (1) selon une des revendications précédentes, disposant également d'une structure de maintien (12) amovible se fixant sur la structure portante (2) et mettant une poignée à disposition du patient.
